# DEMANDE DE BREVET EUROPEEN

(11) **EP 3 346 268 A1**
(43) Date de publication de la demande: **11.07.2018**
(21) Numéro de dépôt: 17306948.5
(22) Date de dépôt: 28.12.2017
(51) Int. Cl.: G01N 33/00

(54) **UNITÉ DE DÉTECTION ET D'ANALYSE, DISPOSITIF DE CONTRÔLE DE PARAMÈTRES PHYSICO-CHIMIQUES D'UN ENVIRONNEMENT COMPRENANT LADITE UNITÉ, ET PROCÉDÉ DE CONTRÔLE ASSOCIÉ**

(30) Priorité: 06.01.2017 FR 1750159
(71) Demandeur: Azimut Monitoring (SAS), 38926 Crolles (FR)
(72) Inventeur: DUFOURNET, Didier, 73190 SAINT JEOIRE PRIEURE (FR); DELACROIX, Alexandre, 38410 SAINT MARTIN D'URIAGE (FR)
(74) Mandataire: Nuss, Laurent

(57) **Abrégé**

La présente invention a pour objet une unité de détection et d'analyse (1) d'une ou plusieurs grandeur(s) physico-chimique(s) comprenant :
un capteur (2, 3) apte et destiné à acquérir des données représentatives d'une ou plusieurs grandeur(s) physico-chimique(s).

L'unité de détection et d'analyse (1) est caractérisée en ce qu'elle comprend en outre :
un module de traitement (4) local apte et destiné à numériser les données représentatives d'une ou plusieurs grandeur(s) physico-chimique(s) provenant dudit au moins un capteur (2, 3) en données numérisées et à mémoriser lesdites données numérisées,
un module de communication (5) comprenant des moyens d'émission et de réception de rayonnements électromagnétiques (6, 7) aptes et destinés à communiquer avec un réseau de communication distant (8') et/ou avec un ou des appareil(s) communicant(s) (9, 9') de régulation d'une ou plusieurs grandeur(s) physico-chimique(s) et/ou une ou des autre(s) unité(s) de détection et d'analyse (1) pour acquérir des données représentatives de l'environnement.

## Description

La présente invention concerne le domaine de l'analyse et du contrôle de la qualité de l'air d'un environnement, et plus particulièrement une unité de détection et d'analyse.

Des capteurs d'analyse de la qualité de l'air permettent, par l'acquisition de grandeurs physico-chimiques, de déterminer la qualité de l'air d'un environnement. Le contrôle de la qualité de l'air répond par ailleurs à des exigences nouvelles liées à la réglementation thermique des bâtiments, à des évolutions des réglementations liées à la ventilation et à une pollution de l'air en milieu urbain croissante. Une bonne qualité de l'air ne peut être assurée qu'en combinant à la fois un bon contrôle et un renouvellement efficace en air neuf, un contrôle de la filtration des polluants extérieurs, liés aux particules et à la pollution photochimique, et à un traitement adapté de la pollution organique engendrée par les activités humaines.

De manière connue pour l'analyse in-situ, les grandeurs physico-chimiques acquises par les capteurs sont restituées, par exemple, par l'intermédiaire d'un afficheur graphique, à un utilisateur qui peut alors établir un diagnostic de la qualité de l'air de l'environnement. Dans le cas où l'environnement consiste en des locaux comprenant une pluralité de pièces, le capteur est déplacé d'une pièce à l'autre et les opérations d'analyse et de diagnostic doivent être réitérées pour chaque pièce afin d'établir un diagnostic pour l'ensemble des locaux. Ces opérations sont particulièrement fastidieuses et les résultats obtenus posent la question de la cohérence temporelle et de la représentativité temporelle, puisque les mesures ne sont pas effectuées simultanément et sont ponctuelles.

Des solutions ont été développées pour tenter d'améliorer ces inconvénients et il a par exemple été proposé de disposer une pluralité de capteurs dans chacune des pièces des locaux et de prévoir une unité centrale délocalisée à laquelle sont communiquées les données brutes relatives aux grandeurs physico-chimiques mesurées par chacun des capteurs. Toutefois, une telle façon de procéder se limite à fournir un diagnostic de la qualité de l'air.

La présente invention a pour but de proposer une solution complète de diagnostic, de régulation et de contrôle de la qualité de l'air d'un environnement permettant de pallier à au moins une des limitations de l'art antérieur.

La présente invention concerne une unité de détection et d'analyse d'une ou plusieurs grandeur(s) physico-chimique(s) comprenant :
au moins un capteur apte et destiné à acquérir des données représentatives d'une ou plusieurs grandeur(s) physico-chimique(s),
unité de détection et d'analyse caractérisée en ce qu'elle comprend en outre :
   au moins un module de traitement local apte et destiné à numériser les données représentatives d'une ou plusieurs grandeur(s) physico-chimique(s) provenant dudit au moins un capteur en données numérisées et à mémoriser lesdites données numérisées,
   au moins un module de communication comprenant des moyens d'émission et de réception de rayonnements électromagnétiques aptes et destinés à communiquer avec un réseau de communication distant et/ou avec un ou des appareil(s) communicant(s) de régulation d'une ou plusieurs grandeur(s) physico-chimique(s) et/ou une ou des autre(s) unité(s) de détection et d'analyse pour acquérir des données représentatives de l'environnement et/ou du fonctionnement de(s)dit(s) un ou des appareil(s) communicant(s),
   en ce que le module de traitement et le module de communication sont reliés entre eux pour permettre au module de communication de transmettre les données représentatives de l'environnement en provenance du réseau de communication distant et/ou un ou des appareil(s) communicant(s) et/ou une ou des autre(s) unité(s) de détection et d'analyse au module de traitement,
   en ce que le module de traitement est apte et destiné à traiter les données numérisées et/ou les données représentatives de l'environnement et/ou du fonctionnement de(s)dit(s) un ou des appareil(s) communicant(s), et/ou de ladite une ou lesdites autre(s) unité(s) de détection et d'analyse pour les transformer en données décisionnelles pour commander le(s)dit(s) un ou des appareil(s) communicant(s) et/ou contrôler leurs états,
   en ce que le module de traitement est apte et destiné à transmettre au module de communication lesdites données décisionnelles,
   en ce que le module de communication est apte et destiné à commander le(s)dit(s) un ou des appareil(s) communicant(s) et/ou ladite une ou lesdites autre(s) unité(s) de détection et d'analyse et/ou contrôler leurs états.

La présente invention concerne également un dispositif de contrôle de paramètres physico-chimiques d'un environnement, caractérisé en ce qu'il comprend :
au moins une unité de détection et d'analyse, telle que décrite précédemment,
au moins un réseau de communication distant, et/ou
au moins un appareil communicant de régulation d'une ou plusieurs grandeur(s) physico-chimique(s).

La présente invention concerne également un procédé de contrôle de paramètres physico-chimiques d'un environnement, caractérisé en ce qu'il met en oeuvre le dispositif de contrôle de paramètres physico-chimiques d'un environnement tel que décrit précédemment, et en ce qu'il comprend au moins :
- une étape de détection et d'analyse, lors de laquelle au moins une unité de détection et d'analyse d'une ou plusieurs grandeur(s) physico-chimique(s), telle que décrite précédemment, comprenant au moins un capteur, acquiert des données représentatives d'une ou plusieurs grandeurs physico-chimiques,
- une première étape de traitement, lors de laquelle un module de traitement local de ladite au moins une unité de détection et d'analyse numérise les données représentatives d'une ou plusieurs grandeur(s) physico-chimique(s) provenant dudit au moins un capteur en données numérisées et mémorise lesdites données numérisées,
- une première étape de communication, lors de laquelle un module de communication de ladite au moins une unité de détection et d'analyse comprenant des moyens d'émission et de réception de rayonnements électromagnétiques communique avec un réseau de communication distant et/ou avec un ou appareil(s) communicant(s) de régulation d'une ou plusieurs grandeur(s) physiques(s) et/ou une ou des autre(s) unité(s) de détection et d'analyse, et acquiert des données représentatives de l'environnement et/ou du fonctionnement de(s)dit(s) un ou des appareil(s) communicant(s), et/ou de ladite ou desdites autre(s) unité(s) de détection et d'analyse,
- une deuxième étape de communication, lors de laquelle le module de communication transmet les données représentatives de l'environnement en provenance du réseau de communication distant et/ou d'un ou des appareil(s) communicant(s) et/ou de ladite ou desdites autre(s) unité(s) de détection et d'analyse au module de traitement, le module de traitement et le module de communication étant reliés entre eux,
- une deuxième étape de traitement, lors de laquelle le module de traitement traite les données numérisées en provenance dudit au moins un capteur et/ou les données représentatives de l'environnement et/ou du fonctionnement de(s)dit(s) un ou des appareil(s) communicant(s), et/ou de ladite ou desdites autre(s) unité(s) de détection et d'analyse et les transforme en données décisionnelles pour commander le(s)dit(s) un ou des appareil(s) communicant et/ou ladite ou lesdites autre(s) unité(s) de détection et d'analyse et/ou pour contrôler leurs états,
- une troisième étape de communication, lors de laquelle le module de traitement transmet au module de communication lesdites données décisionnelles,
- une étape de commande, lors de laquelle le module de communication commande le(s)dit(s) un ou des appareil(s) communicant(s) et/ou ladite ou lesdites autre(s) unité(s) de détection et d'analyse et/ou contrôle leurs états à partir desdites données décisionnelles.

L'invention sera mieux comprise, grâce à la description ci-après, qui se rapporte à plusieurs modes de réalisation préférés, donnés à titre d'exemples non limitatifs, et expliqués avec référence aux dessins schématiques annexés, dans lesquels :

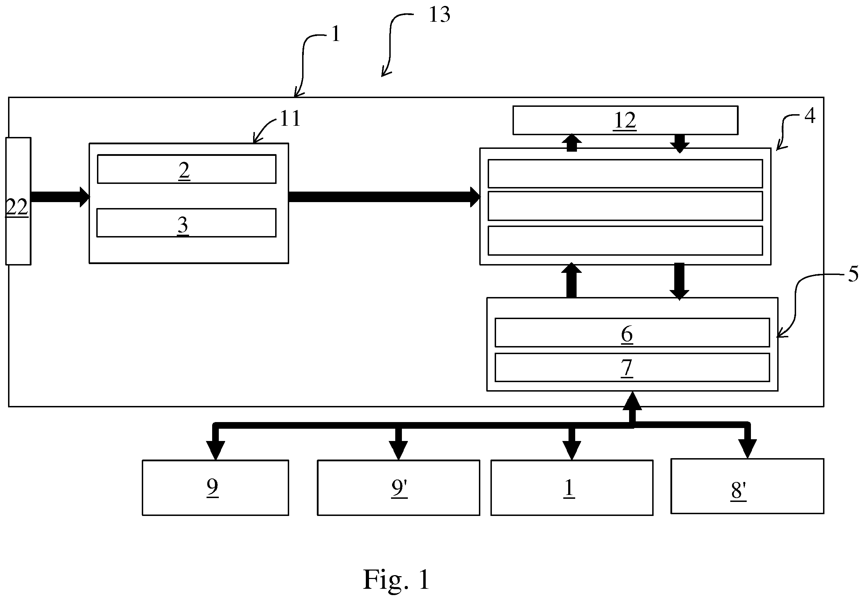

la figure 1 est une vue schématique du dispositif de contrôle selon l'invention, et
la figure 2 est une vue schématique de l'implantation d'un dispositif de contrôle selon l'invention dans des locaux.

L'unité de détection et d'analyse 1 d'une ou plusieurs grandeur(s) physico-chimique(s) comprend :
au moins un capteur apte et destiné à acquérir des données représentatives d'une ou plusieurs grandeur(s) physico-chimique(s).

Conformément à l'invention, l'unité de détection et d'analyse 1 est caractérisée en ce qu'elle comprend en outre :
au moins un module de traitement 4 local apte et destiné à numériser les données représentatives d'une ou plusieurs grandeur(s) physico-chimique(s) provenant dudit au moins un capteur en données numérisées et à mémoriser lesdites données numérisées,
au moins un module de communication 5 comprenant des moyens d'émission et de réception de rayonnements électromagnétiques aptes et destinés à communiquer, préférentiellement par l'intermédiaire d'un lien de communication 16, 17, 18, 19, 20, 21, par exemple bidirectionnel, avec un réseau de communication distant 8, 8', 8" et/ou avec un ou des appareil(s) communicant(s) 9, 9', 10 de régulation d'une ou plusieurs grandeur(s) physico-chimique(s) et/ou une ou des autre(s) unité(s) de détection et d'analyse 1 pour acquérir des données représentatives de l'environnement et/ou du fonctionnement de(s)dit(s) un ou des appareil(s) communicant(s) 9, 9', 10,
en ce que le module de traitement 4 et le module de communication 5 sont reliés entre eux pour permettre au module de communication 5 de transmettre les données représentatives de l'environnement en provenance du réseau de communication distant 8, 8', 8" et/ou un ou des appareil(s) communicant(s) 9, 9', 10 et/ou une ou des autre(s) unité(s) de détection et d'analyse 1 au module de traitement 4,
en ce que le module de traitement 4 est apte et destiné à traiter les données numérisées et/ou les données représentatives de l'environnement et/ou du fonctionnement de(s)dit(s) un ou des appareil(s) communicant(s) 9, 9', 10 et/ou de ladite une ou lesdites autre(s) unité(s) de détection et d'analyse 1 pour les transformer en données décisionnelles pour commander le(s)dit(s) un ou des appareil(s) communicant(s) 9, 9', 10 et/ou contrôler leurs états,
en ce que le module de traitement 4 est apte et destiné à transmettre au module de communication 5 lesdites données décisionnelles,
en ce que le module de communication 5 est apte et destiné à commander le(s)dit(s) un ou des appareil(s) communicant(s) 9, 9',10 et/ou ladite une ou lesdites autre(s) unité(s) de détection et d'analyse 1 et/ou contrôler leurs états à partir des données décisionnelles.

Avantageusement, l'unité de détection et d'analyse 1 selon l'invention a la faculté de traiter de façon locale les données représentatives d'une ou plusieurs grandeur(s) physico-chimique(s) provenant de son ou de ses capteur(s) 2, 3 et les données représentatives de l'environnement issues d'un réseau de communication distant 8, 8', 8", et/ou des appareils communicants 9, 9', 10 et/ou d'autres unités de détection et d'analyse 1. L'unité de détection et d'analyse 1 peut délivrer par la suite des informations enrichies sous la forme de données décisionnelles. Il est ainsi possible de s'affranchir de l'utilisation d'une unité centrale délocalisée, comme c'est le cas dans l'art antérieur, à laquelle seraient transmises les données représentatives d'une ou plusieurs grandeur(s) physico-chimique(s) provenant d'un capteur séparé qui sont des données brutes, c'est-à-dire qui n'ont pas subies de traitement. La sécurité et la qualité du service sont par ailleurs améliorées puisque les données décisionnelles ne doivent pas obligatoirement être transmises au réseau de communication distant 8, 8', 8". En outre, seules des données décisionnelles sont communiquées aux appareils communicants 9, 9', 10 de régulation d'une ou plusieurs grandeur(s) physico-chimique(s) ou aux autres unités de détection et d'analyse 1 ou au réseau de communication distant 8, 8', 8". On évite ainsi de surcharger les liens de communication 16, 17, 18, 19, 20, 21 et de saturer la mémoire d'une unité centrale délocalisée, comme c'est le cas dans l'art antérieur.

Le module de traitement 4 local peut non seulement permettre de numériser les données représentatives d'une ou plusieurs grandeur(s) physico-chimique(s) provenant dudit au moins un capteur en données numérisées et à mémoriser lesdites données numérisées, mais il peut également permettre la fusion des données numérisées provenant des données représentatives d'une ou plusieurs grandeur(s) physico-chimique(s) provenant dudit au moins un capteur 2, 3. En d'autres termes, le module de traitement 4 peut comparer les données numérisées pour fusionner ces données et ainsi fournir une interprétation locale de ces données qui sont issues de grandeurs mesurées multiples. Il en résulte, que le module de traitement 4 peut fournir une information enrichie de haut niveau permettant une gestion intelligente locale, c'est-à-dire au niveau de l'unité de détection et d'analyse 1. Par exemple, il est ainsi possible d'effectuer des correction d'une grandeur physico-chimique, par exemple un taux de particules fines, en fonction d'une autre ou d'autres grandeur(s) physico-chimique(s), par exemple la pression et/ou le taux d'humidité.

Le module de traitement 4 peut comprendre au moins une règle d'inférence pour permettre le traitement des données numérisées en provenance dudit au moins un capteur 2, 3 et/ou des données numérisées fusionnées en provenance dudit au moins un capteur 2, 3, et/ou les données représentatives de l'environnement et/ou du fonctionnement de(s)dit(s) un ou des appareil(s) communicant(s) 9, 9', 10 et/ou de ladite ou desdites autre(s) unité(s) de détection et d'analyse 1. En effet, les données numérisées en provenance dudit au moins un capteur 2, 3 et/ou les données numérisées fusionnées en provenance dudit au moins un capteur 2, 3, et/ou les données représentatives de l'environnement et/ou du fonctionnement de(s)dit(s) un ou des appareil(s) communicant(s) 9, 9', 10 et/ou de ladite ou desdites autre(s) unité(s) de détection et d'analyse 1 forment les paramètres d'au moins une règle d'inférence, préférentiellement prédéterminée dont la conclusion correspond aux données décisionnelles.

De cette façon, le module de traitement 4 transforme les données en provenance du capteur 2, 3 en informations exploitables, par exemple des informations sur la calibration, sur des coefficients de corrélation, de préférence à l'aide d'algorithmes spécifiques. Le module de traitement 4 local intègre également les données issues de son environnement pour décider de la meilleure consigne à appliquer. Le module de traitement 4 peut alors décider des actions à envoyer notamment aux appareil(s) communicant(s) 9, 9', 10, de type centrale de traitement d'air et/ou de type dispositif de purification et/ou de filtration d'air 10 pour atteindre la consigne donnée.

Les grandeurs physico-chimiques peuvent être choisies parmi : la température, la pression, l'hygrométrie, une teneur en gaz, par exemple, polluants photo-oxydants, une teneur en composés organiques volatiles, une teneur en dioxyde de carbone, une teneur en particules et en aérosols, une teneur en gaz avec composantes soufrées. Bien entendu, ces exemples ne sont pas limitatifs.

Les données représentatives de l'environnement peuvent consister en des données climatiques, en des données caractérisant la pollution de l'air extérieur, en des données qualifiant l'occupation des locaux, en des données issues de paramètres et de contraintes de gestion technique du bâtiment, en des données qualifiant les différentes activités humaines présentes dans le bâtiment.

Les données décisionnelles peuvent consister en un ordre de mise en marche, d'arrêt ou de régulation des appareils communicants 9, 9', 10.

L'unité de détection et d'analyse 1 peut comprendre un module d'analyse de l'air 11 comprenant ledit au moins un capteur choisi seul ou en combinaison parmi un capteur optique 2, un capteur de détection physico-chimique de l'air 3.

De préférence, le module d'analyse de l'air 11 comprend une pluralité de capteurs.

Le capteur optique 2 peut être basé sur des technologies optiques en lumière du spectre visible ou non, ou sur des technologies de type photo-acoustiques.

Le capteur de détection physico-chimique 3 peut être un capteur électrochimique, ou un capteur de technologie semi-conducteur.

Le capteur optique 2 et/ou le capteur de détection physico-chimique 3 pourront être basés sur des technologies classiques, de type mini capteurs, ou sur des technologies à forte intégration de forme, de type MEMS ou MOEMS, de tels capteurs présentent l'avantage d'être miniaturisés puisque leurs dimensions peut être de l'ordre quelques millimètres cubes.

Préférentiellement, le capteur 2, 3 est sous la forme d'un MEMS, permettant de préférence la mesure de la température et/ou l'humidité et/ou la pression et/ou du taux de dioxyde de carbone et/ou du taux de dioxyde d'azote et/ou du taux de composés organiques volatiles et/ou de la concentration de particules fines. Un capteur 2, 3 sous la forme de MEMS peut de manière optionnelle permettre la fusion des données numérisées provenant des données représentatives d'une ou plusieurs grandeur(s) physico-chimique(s) mesurées par ce capteur 2, 3 MEMS. Un exemple d'un tel capteur 2, 3 est connu et commercialisé sous la dénomination Bosch BME680 (Marque déposée).

Le ou les capteur(s) 2, 3 peu(vent) être passif(s) ou actif(s).

L'unité de détection et d'analyse 1 peut comprendre une admission d'air 22 passive ou active pour permettre l'écoulement de l'air jusqu'au capteur optique 2 et /ou au capteur de détection physico-chimique 3 du module d'analyse de l'air 11.

Le module de traitement 4 peut comprendre une mémoire 12 dans laquelle les différentes données peuvent être stockées.

Le module de traitement 4 peut comprendre un processeur, ou un microprocesseur.

Les moyens d'émission et de réception de rayonnements électromagnétiques du module de communication 5 peuvent être choisis seul(s) ou en combinaison parmi des moyens de liaison locale sans fil 6, des moyens de liaison sans fil à faible consommation énergétique 7 pour établir des liens de communication 16, 17, 18, 19, 20, 21, préférentiellement bidirectionnels.

La liaison locale sans fil 6 peut être destinée à une communication locale intra-locaux 14, par exemple utilisant Zigbee, Wifi et ses variantes, Bluetooth et ses variantes, KNX-radio, ou analogue.

La liaison dans fil 7 peut être destinée à une communication distante extra-locaux 14, par exemple, réseaux opérés comme LoRa, Sigfox, NBIoT, GPRS, 4G, ou analogue.

Le module de communication 5 peut comprendre des moyens pour interroger à intervalles de temps, de préférence régulier, le réseau de communication distant 8, 8', 8" et/ou un ou des appareil(s) communicant(s) 9, 9', 10 et/ou une ou des autre(s) unité(s) de détection et d'analyse 1.

L'unité de détection et d'analyse 1 peut être entourée d'un boîtier (non représenté).

Par exemple, ce boîtier peut être portatif ou fixe. Dans ce cas, le boîtier peut comprendre des moyens de fixation pour être fixé à un support, tel qu'un mur ou un plafond.

Le boîtier peut prendre la forme d'un interrupteur ou d'un détecteur de fumée et/ou de présence.

L'unité de détection et d'analyse 1 peut comprendre un afficheur graphique (non représenté) permettant d'afficher différentes informations visibles par un utilisateur.

Conformément à l'invention, le dispositif de contrôle 13 de paramètres physico-chimiques d'un environnement, est caractérisé en ce qu'il comprend :
au moins une unité de détection et d'analyse 1 telle que décrite précédemment,
au moins un réseau de communication distant 8, 8', 8", et/ou
au moins un appareil communicant 9, 9', 10 de régulation d'une ou plusieurs grandeur(s) physico-chimique(s).

Avantageusement, le dispositif de contrôle 13 forme un réseau comprenant au moins une unité de détection et d'analyse 1, au moins un réseau de communication distant 8, 8', 8", et/ou au moins un appareil communicant 9, 9', 10 de régulation d'une ou plusieurs grandeur(s) physico-chimique(s).

Le dispositif de contrôle 13 peut comprendre des unités de détection et d'analyse 1 pouvant être identiques.

Un ou des appareil(s) communicant(s) de régulation d'une ou plusieurs grandeur(s) physico-chimique(s) peu(ven)t être choisi(s) seul(s) ou en combinaison parmi un dispositif de chauffage et/ou de ventilation et/ou de climatisation 9, un dispositif de purification et/ou de filtration d'air 10, un ouvrant (non représenté), un système d'automatisation d'un bâtiment 9', un dispositif de contrôle de la régulation thermique et hydrique (non représenté), ainsi que tout équipement influant sur le confort et la qualité d'air de l'occupant.

De préférence, l'appareil communicant 9, 9', 10 peut être un équipement de régulation du confort et de la qualité de l'air, tel qu'une centrale de traitement de l'air et/ou un purificateur d'air.

Un ou des réseau(x) de communication distant(s) peu(ven)t être choisi seul(s) ou en combinaison parmi un réseau internet 8, un ou des serveurs externes 8', une station météorologique 8".

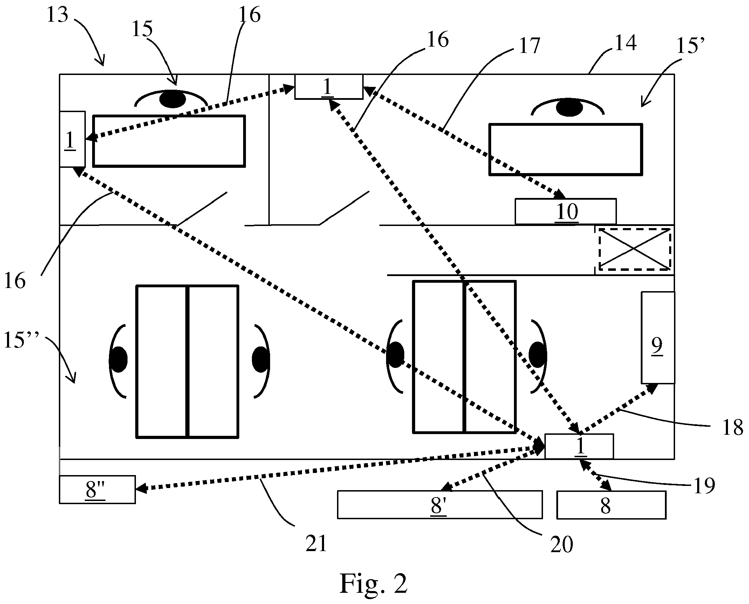

Le ou les unité(s) de détection et d'analyse 1 et le ou les appareil(s) communicant(s) 9, 9', 10 peu(ven)t être disposé(s) à l'intérieur de locaux 14 et le ou les réseau(x) de communication distant(s) 8, 8', 8" peu(ven)t être disposé à l'extérieur des locaux 14 (figure 2).

Les locaux 14 peuvent être, à titre d'exemples non limitatifs, un bâtiment publique, un hôtel, des bureaux, des commerces, des administrations, des hôpitaux, un logement individuel ou collectif.

Comme l'illustre la figure 2, les locaux 14 peuvent comprendre plusieurs pièces 15, 15', 15" comprenant chacune une unité de détection et d'analyse 1. Une de ces pièces 15' peut comprendre un dispositif de purification et/ou de filtration d'air 10. Une autre de ces pièces 15" peut comprendre un dispositif de chauffage et/ou de ventilation et/ou de climatisation 9. En outre, à l'extérieur des locaux 14 peuvent être disposés un réseau internet 8, un ou des serveur(s) externe(s) 8', et une station météorologique 8".

Ces unités de détection et d'analyse 1 peuvent établir des liens de communication bidirectionnels 16 les unes entre les autres. Avantageusement, les unités de détection et d'analyse 1 peuvent échanger leurs données décisionnelles.

L'unité de détection et d'analyse 1 de la pièce 15' comprenant le dispositif de purification et/ou de filtration d'air 10 peut également établir un lien de communication bidirectionnel 17 avec le dispositif de purification et/ou de filtration d'air 10. Avantageusement, l'unité de détection et d'analyse 1 peut transmettre un ordre de marche ou d'arrêt ou de niveau de régulation au dispositif de purification et/ou de filtration d'air 10. Réciproquement, le dispositif de purification et/ou de filtration d'air 10 peut transmettre des données représentatives de son état de fonctionnement à l'unité de détection et d'analyse 1.

L'unité de détection et d'analyse 1 de la pièce 15" comprenant le dispositif de chauffage et/ou de ventilation et/ou de climatisation 9 peut également établir un lien de communication bidirectionnel 18 avec le dispositif de chauffage et/ou de ventilation et/ou de climatisation 9. Avantageusement, l'unité de détection et d'analyse 1 peut transmettre un ordre de marche ou d'arrêt ou de niveau de régulation au dispositif de chauffage et/ou de ventilation et/ou de climatisation 9. Réciproquement, le dispositif de chauffage et/ou de ventilation et/ou de climatisation 9 peut transmettre des données représentatives de son état de fonctionnement à l'unité de détection et d'analyse 1.

Cette unité de de détection et d'analyse 1 de la pièce 15" peut également établir un lien de communication bidirectionnel 19 avec un réseau internet 8, un lien de communication bidirectionnel 20 avec un ou des serveurs externes 8', un lien de communication bidirectionnel 21 avec une station météorologique 8". Avantageusement, l'unité de détection et d'analyse 1 peut transmettre des informations sur son état de fonctionnement à des utilisateurs distants ou l'unité de détection et d'analyse 1 peut réceptionner des informations météorologiques de la station météorologique 8".

Conformément à l'invention, le procédé de contrôle de paramètres physico-chimiques d'un environnement, est caractérisé en ce qu'il met en oeuvre le dispositif de contrôle 13 de paramètres physico-chimiques d'un environnement tel que décrit précédemment, et en ce qu'il comprend au moins :
- une étape de détection et d'analyse, lors de laquelle au moins une unité de détection et d'analyse 1 d'une ou plusieurs grandeur(s) physico-chimique(s) telle que décrite précédemment, comprenant au moins un capteur 2, 3, acquiert des données représentatives d'une ou plusieurs grandeur(s) physico-chimique(s),
- une première étape de traitement, lors de laquelle un module de traitement 4 local de ladite au moins une unité de détection et d'analyse 1 numérise les données représentatives d'une ou plusieurs grandeur(s) physico-chimique(s) provenant dudit au moins un capteur 2, 3 en données numérisées et mémorise lesdites données numérisées,
- une première étape de communication, lors de laquelle un module de communication 5 de ladite au moins une unité de détection et d'analyse 1 comprenant des moyens d'émission et de réception de rayonnements électromagnétiques 6, 7 communique avec un réseau de communication distant 8, 8', 8" et/ou avec un ou des appareil(s) communicant(s) 9, 9', 10 de régulation d'une ou plusieurs grandeur(s) physico-chimique(s) et/ou une ou des autre(s) unité(s) de détection et d'analyse 1, et acquiert des données représentatives de l'environnement et/ou du fonctionnement de(s)dit(s) un ou des appareil(s) communicant(s) 9, 9',10 et/ou de ladite ou desdites autre(s) unité(s) de détection et d'analyse 1,
- une deuxième étape de communication, lors de laquelle le module de communication 5 transmet les données représentatives de l'environnement en provenance du réseau de communication distant 8, 8', 8" et/ou d'un ou des appareil(s) communicant(s) 9, 9', 10 et/ou de ladite ou desdites autre(s) unité(s) de détection et d'analyse 1 au module de traitement 4, le module de traitement 4 et le module de communication 5 étant reliés entre eux,
- une deuxième étape de traitement, lors de laquelle le module de traitement 4 traite les données numérisées en provenance dudit au moins un capteur 2, 3 et/ou les données représentatives de l'environnement et/ou du fonctionnement de(s)dit(s) un ou des appareil(s) communicant(s) 9, 9', 10 et/ou de ladite ou desdites autre(s) unité(s) de détection et d'analyse 1 et les transforme en données décisionnelles pour commander le(s)dit(s) un ou des appareil(s) communicant(s) 9, 9', 10 et/ou ladite ou lesdites autre(s) unité(s) de détection et d'analyse 1 et/ou pour contrôler leurs états,
- une troisième étape de communication, lors de laquelle le module de traitement 4 transmet au module de communication 5 lesdites données décisionnelles,
- une étape de commande, lors de laquelle le module de communication 5 commande le(s)dit(s) un ou des appareil(s) communicant(s) 9, 9', 10 et/ou ladite ou lesdites autre(s) unité(s) de détection et d'analyse 1 et/ou contrôle leurs états à partir desdites données décisionnelles.

Lors de la première étape de traitement, les données représentatives d'une ou plusieurs grandeur(s) physico-chimique(s) peuvent être acquises au moyen dudit au moins un capteur 2, 3.

Lors de la première étape de traitement, le module de traitement 4 peut mémoriser les données dans une mémoire 12 du module de traitement 4.

Lors de l'étape de détection et d'analyse, ladite au moins une unité de détection et d'analyse 1 peut comprendre un module d'analyse de l'air 11 comprenant au moins un capteur choisi seul ou en combinaison parmi un capteur optique 2, un capteur de détection physico-chimique de l'air 3, acquiert les données représentatives d'une ou plusieurs grandeur(s) physico-chimique(s) relatives à la qualité de l'air à l'aide dudit au moins un capteur.

Lors d'une quatrième étape de communication, le module de communication 5 peut communiquer lesdites données décisionnelles au réseau de communication distant 8, 8', 8" et/ou audit un ou des appareil(s) communicant(s) 9, 9', 10.

Lors d'une étape de contrôle ultérieure à l'étape de commande, l'étape de détection et d'analyse peut être réitérée pour acquérir des nouvelles données représentatives d'une ou plusieurs grandeur(s) physico-chimique(s). Puis, lors d'une troisième étape de traitement, ces nouvelles données sont comparées par le module de traitement 4 à un critère décisionnel, puis transformées en nouvelles données pour commander un ou des appareil(s) communicant(s) 9, 9', 10. En outre, si les nouvelles données représentatives d'une ou plusieurs grandeur(s) physico-chimique(s) répondent à un critère décisionnel, alors l'étape de commande peut être réitérée.

Par exemple, lorsque l'appareil communicant est un dispositif de purification et/ou de filtration d'air 10, lors de l'étape de commande, l'unité de détection et d'analyse 1 communique lesdites données décisionnelles sous la forme d'un ordre de niveau de régulation au dispositif de purification et/ou de filtration d'air 10. Puis, lors de l'étape de contrôle l'unité de détection et d'analyse 1 acquiert des nouvelles données représentatives d'une ou plusieurs grandeur(s) physico-chimique(s). Ces nouvelles données peuvent être comparées à un critère décisionnel par le module de traitement 4 qui peut les transformer en nouvelles données décisionnelles pour commander le dispositif de purification et/ou de filtration d'air 10. Si les nouvelles données répondent à un critère décisionnel, alors le module de communication 5 peut commander le niveau de régulation du dispositif de purification et/ou de filtration d'air 10.

Lors de la deuxième étape de traitement, les données numérisées en provenance dudit au moins un capteur 2, 3 et/ou les données représentatives de l'environnement et/ou du fonctionnement de(s)dit(s) un ou des appareil(s) communicant(s) 9, 9', 10 et/ou de ladite ou desdites autre(s) unité(s) de détection et d'analyse 1 peuvent former les paramètres d'au moins une règle d'inférence, préférentiellement prédéterminée dont la conclusion correspond aux données décisionnelles.

La règle d'inférence correspond à une règle logique qui peut comprendre un ensemble de conditions dont le résultat ou la conclusion sous la forme de données décisionnelles peut être une action. Par exemple, la règle d'inférence peut être : SI condition_1 ET condition_2 ETPAS condition_3 ALORS déclenchement du signal_1. La règle d'inférence peut être prédéterminée ou peut alternativement être issue d'algorithme d'apprentissage in situ.

Avantageusement, lors de la deuxième étape de traitement et/ou de la troisième étape de traitement, les données numérisées en provenance dudit au moins un capteur 2, 3 et/ou les données représentatives de l'environnement et/ou du fonctionnement de(s)dit(s) un ou des appareil(s) communicant(s) 9, 9', 10 et/ou de ladite ou desdites autre(s) unité(s) de détection et d'analyse 1 sont fusionnées pour former des données décisionnelles qui ne sont pas brutes, mais agrégées, puisqu'elles sont issues de plusieurs sources, et qui sont renforcées, puisque la décision peut être prise avec un haut niveau de confiance. Par ailleurs, les données collectées peuvent être concordantes ou concurrentes, les règles d'inférences, préférentiellement prédéterminées peuvent tenir compte de ces facteurs tendanciels pour influer sur les données décisionnelles. Des données concordantes peuvent par exemple consister en une information sur un taux de dioxyde carbone relevé qui est élevé dans l'ensemble des pièces 15, 15', 15" des locaux 14. Des données concurrentes peuvent par exemple consister en une information sur un niveau de particules relevé pour une pièce 15 qui est élevé et pour une autre pièce 15' qui est inférieur au niveau de la pièce 15.

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Unité de détection et d'analyse (1) d'une ou plusieurs grandeur(s) physico-chimique(s) comprenant :
au moins un capteur (2, 3) apte et destiné à acquérir des données représentatives d'une ou plusieurs grandeur(s) physico-chimique(s),
unité de détection et d'analyse (1) **caractérisée en ce qu'**elle comprend en outre :
au moins un module de traitement (4) local apte et destiné à numériser les données représentatives d'une ou plusieurs grandeur(s) physico-chimique(s) provenant dudit au moins un capteur (2, 3) en données numérisées et à mémoriser lesdites données numérisées,
au moins un module de communication (5) comprenant des moyens d'émission et de réception de rayonnements électromagnétiques (6, 7) aptes et destinés à communiquer avec un réseau de communication distant (8, 8', 8") et/ou avec un ou des appareil(s) communicant(s) (9, 9', 10) de régulation d'une ou plusieurs grandeur(s) physico-chimique(s) et/ou une ou des autre(s) unité(s) de détection et d'analyse (1) pour acquérir des données représentatives de l'environnement et/ou du fonctionnement de(s)dit(s) un ou des appareil(s) communicant(s) (9, 9', 10),
**en ce que** le module de traitement (4) et le module de communication (5) sont reliés entre eux pour permettre au module de communication (5) de transmettre les données représentatives de l'environnement en provenance du réseau de communication distant (8, 8', 8") et/ou un ou des appareil(s) communicant(s) (9, 9', 10) et/ou une ou des autre(s) unité(s) de détection et d'analyse (1) au module de traitement (4),
**en ce que** le module de traitement (4) est apte et destiné à traiter les données numérisées et/ou les données représentatives de l'environnement et/ou du fonctionnement de(s)dit(s) un ou des appareil(s) communicant(s) (9, 9', 10) et/ou de ladite une ou lesdites autre(s) unité(s) de détection et d'analyse (1) pour les transformer en données décisionnelles pour commander le(s)dit(s) un ou des appareil(s) communicant(s) (9, 9', 10) et/ou contrôler leurs états,
**en ce que** le module de traitement (4) est apte et destiné à transmettre au module de communication (5) lesdites données décisionnelles,
**en ce que** le module de communication (5) est apte et destiné à commander le(s)dit(s) un ou des appareil(s) communicant(s) (9, 9', 10) et/ou ladite une ou lesdites autre(s) unité(s) de détection et d'analyse (1) et/ou contrôler leurs états.

2. Unité de détection et d'analyse selon la revendication 1, **caractérisée en ce que** le module de traitement (4) local est apte et destiné à permettre la fusion des données numérisées provenant des données représentatives d'une ou plusieurs grandeur(s) physico-chimique(s) provenant dudit au moins un capteur (2, 3).

3. Unité de détection et d'analyse l'une quelconque des revendications 1 à 2, **caractérisée en ce que** le module de communication (5) est apte et destiné à commander un appareil communicant (9, 9', 10) de régulation d'une ou plusieurs grandeur(s) physico-chimique(s), préférentiellement un dispositif de purification et/ou de filtration d'air (10).

4. Unité de détection et d'analyse selon l'une quelconque des revendications 1 à 3, **caractérisé en ce qu'**elle comprend un module d'analyse de l'air (11) comprenant ledit au moins un capteur choisi seul ou en combinaison parmi un capteur optique (2), un capteur de détection physico-chimique de l'air (3).

5. Unité de détection et d'analyse selon la revendication 4, **caractérisé en ce que** le capteur (2, 3) est sous la forme d'un MEMS.

6. Unité de détection et d'analyse selon l'une quelconque des revendications 1 à 5, **caractérisé en ce que** le module de traitement (4) comprend une mémoire (12).

7. Unité de détection et d'analyse selon l'une quelconque des revendications 1 à 6, **caractérisé en ce que** les moyens d'émission et de réception de rayonnements électromagnétiques du module de communication (5) sont choisis seul(s) ou en combinaison parmi des moyens de liaison locale sans fil (6), des moyens de liaison sans fil à faible consommation énergétique (7).

8. Unité de détection et d'analyse selon l'une quelconque des revendications 1 à 7, l'unité de détection et d'analyse (1) est entourée d'un boîtier.

9. Dispositif de contrôle (13) de paramètres physico-chimiques d'un environnement, **caractérisé en ce qu'**il comprend :
au moins une unité de détection et d'analyse (1) selon l'une quelconque des revendications 1 à 8,
au moins un réseau de communication distant (8, 8', 8"), et/ou
au moins un appareil communicant (9, 9', 10) de régulation d'une ou plusieurs grandeur(s) physico-chimique(s).

10. Dispositif selon la revendication 9, **caractérisé en ce qu'**un ou des appareil(s) communicant(s) sont choisi(s) seul(s) ou en combinaison parmi un dispositif de chauffage et/ou de ventilation et/ou de climatisation (9), un dispositif de purification et/ou de filtration d'air (10), un ouvrant, un système d'automatisation d'un bâtiment (9').

11. Dispositif selon l'une quelconque des revendications 9 à 10, le réseau de communication distant est choisi parmi un réseau internet (8), un ou des serveurs externes (8'), une station météorologique (8").

12. Dispositif selon l'une quelconque des revendications 9 à 11, **caractérisé en ce que** l'unité de détection et d'analyse (1) et les appareil(s) communicant(s) (9, 9', 10) sont disposés à l'intérieur de locaux (14) et **en ce que** le réseau de communication distant (8) est disposé à l'extérieur des locaux (14).

13. Procédé de contrôle de paramètres physico-chimiques d'un environnement, **caractérisé en ce qu'**il met en oeuvre le dispositif de contrôle (13) de paramètres physico-chimiques d'un environnement selon l'une quelconque des revendications 9 à 12, et **en ce qu'**il comprend au moins :
- une étape de détection et d'analyse, lors de laquelle au moins une unité de détection et d'analyse (1) d'une ou plusieurs grandeur(s) physico-chimique(s) selon l'une quelconque des revendications 1 à 8, comprenant au moins un capteur (2, 3), acquiert des données représentatives d'une ou plusieurs grandeur(s) physico-chimique(s),
- une première étape de traitement, lors de laquelle un module de traitement (4) local de ladite au moins une unité de détection et d'analyse (1) numérise les données représentatives d'une ou plusieurs grandeur(s) physico-chimique(s) provenant dudit au moins un capteur (2, 3) en données numérisées et mémorise lesdites données numérisées,
- une première étape de communication, lors de laquelle un module de communication (5) de ladite au moins une unité de détection et d'analyse (1) comprenant des moyens d'émission et de réception de rayonnements électromagnétiques (6, 7) communique avec un réseau de communication distant (8, 8', 8") et/ou avec un ou des appareil(s) communicant(s) (9, 9', 10) de régulation d'une ou plusieurs grandeur(s) physico-chimique(s) et/ou une ou des autre(s) unité(s) de détection et d'analyse (1), et acquiert des données représentatives de l'environnement et/ou du fonctionnement de(s)dit(s) un ou des appareil(s) communicant(s) (9, 9',10) et/ou de ladite ou desdites autre(s) unité(s) de détection et d'analyse (1),
- une deuxième étape de communication, lors de laquelle le module de communication (5) transmet les données représentatives de l'environnement en provenance du réseau de communication distant (8, 8', 8") et/ou d'un ou des appareil(s) communicant(s) (9, 9', 10) et/ou de ladite ou desdites autre(s) unité(s) de détection et d'analyse (1) au module de traitement (4), le module de traitement (4) et le module de communication (5) étant reliés entre eux,
- une deuxième étape de traitement, lors de laquelle le module de traitement (4) traite les données numérisées en provenance dudit au moins un capteur (2, 3) et/ou les données représentatives de l'environnement et/ou du fonctionnement de(s)dit(s) un ou des appareil(s) communicant(s) (9, 9', 10) et/ou de ladite ou desdites autre(s) unité(s) de détection et d'analyse (1) et les transforme en données décisionnelles pour commander le(s)dit(s) un ou des appareil(s) communicant(s) (9, 9', 10) et/ou ladite ou lesdites autre(s) unité(s) de détection et d'analyse (1) et/ou pour contrôler leurs états,
- une troisième étape de communication, lors de laquelle le module de traitement (4) transmet au module de communication (5) lesdites données décisionnelles,
- une étape de commande, lors de laquelle le module de communication (5) commande le(s)dit(s) un ou des appareil(s) communicant(s) (9, 9', 10) et/ou ladite ou lesdites autre(s) unité(s) de détection et d'analyse (1) et/ou contrôle leurs états à partir desdites données décisionnelles.

14. Procédé selon la revendication 13, **caractérisé en ce que** lors de la première étape de traitement, le module de traitement (4) mémorise les données dans une mémoire (12) du module de traitement (4).

15. Procédé selon l'une quelconque des revendications 13 à 14, **caractérisé en ce que** lors de l'étape de détection et d'analyse, ladite au moins une unité de détection et d'analyse (1) qui comprend un module d'analyse de l'air (11) comprenant au moins un capteur choisi seul ou en combinaison parmi un capteur optique (2), un capteur de détection physico-chimique de l'air (3), acquiert les données représentatives d'une ou plusieurs grandeur(s) physico-chimique(s) relatives à la qualité de l'air à l'aide dudit au moins un capteur.

16. Procédé selon l'une quelconque des revendications 13 à 15, **caractérisé en ce que** lors d'une quatrième étape de communication, le module de communication (5) communique lesdites données décisionnelles au réseau de communication distant (8, 8', 8") et/ou audit un ou des appareil(s) communicant(s) (9, 9', 10).

17. Procédé selon l'une quelconque des revendications 13 à 16, **caractérisé en ce que** lors d'une étape de contrôle ultérieure à l'étape de commande, l'étape de détection et d'analyse est réitérée pour acquérir des nouvelles données représentatives d'une ou plusieurs grandeur(s) physico-chimique(s), lors d'une troisième étape de traitement ces nouvelles données sont comparées par le module de traitement (4) à un critère décisionnel, puis transformées en nouvelles données décisionnelles pour commander un ou des appareil(s) communicant(s) (9, 9', 10).

18. Procédé selon l'une quelconque des revendications 13 à 17, **caractérisé en ce que** lors de la deuxième étape de traitement et/ou de la troisième étape de traitement, les données numérisées en provenance dudit au moins un capteur (2, 3) et/ou les données représentatives de l'environnement et/ou du fonctionnement de(s)dit(s) un ou des appareil(s) communicant(s) (9, 9', 10) et/ou de ladite ou desdites autre(s) unité(s) de détection et d'analyse (1) forment les paramètres d'au moins une règle d'inférence, préférentiellement prédéterminée, dont la conclusion correspond aux données décisionnelles.
